# FASCICULE DE BREVET EUROPEEN

(11) **EP 0 445 843 B1**
(45) Date de publication et mention de la délivrance du brevet: **09.02.1994**
(21) Numéro de dépôt: 91105956.6
(22) Date de dépôt: 19.11.1987
(51) Int. Cl.: C07C 309/63, C07F 9/09, C07F 9/6574, C07C 69/76

(54) **Nouveau procédé de préparation de dérivés de l'acide cyclopropane carboxylique portant en 3 une chaîne halogénée saturée**
Verfahren zur Herstellung von in Stellung 3 durch eine halogenierte gesättigte Kette substituierten Cyclopropancarbonsäurederivaten
Process for the preparation of cyclopropanecarboxylic acid derivatives substituted at position 3 by a saturated halogenated chain

(30) Priorité: 20.11.1986 FR 8616155
(43) Date de publication de la demande: 11.09.1991
(62) Demande divisionnaire de: 87402605.7
(73) Titulaire: ROUSSEL-UCLAF, 75007 Paris (FR)
(72) Inventeur: Cadiergue, Joseph, F-93600 Aulnay Sous Bois (FR); Demoute, Jean-Pierre, F-93360 Neuilly Plaisance (FR); Tessier, Jean, F-94300 Vincennes (FR)
(74) Mandataire: Tonnellier, Marie-José

(56) Documents cités:
- EP-A- 0 187 674
- CHEMICAL ABSTRACTS, vol. 86, no. 19, 9 mai 1977, page 513, résumé no. 139480a,Columbus, Ohio, US; & JP-A-76 122 041
- TETRAHEDRON LETTERS, vol. 27, no. 19, 1986, pages 2139-2142, Pergamon Journals, Ltd., GB; M. FUJITA et al, "Practical and stereocontrolled syntheses of both (1R*, 3S*)- and (1R*,3R*)-3-(2-chloro-3,3,3-trifluoro-1-propenyl)-2,2-dimethylcyclopropanecarboxylates"

## Description

La présente invention concerne un nouveau procédé de préparation de dérivés de l'acide 2,2-diméthyl cyclopropane carboxylique portant en 3 une chaîne halogénée saturée.

On connaissait des procédés de préparation de carbinols de formule I pouvant être schématisés comme suit :

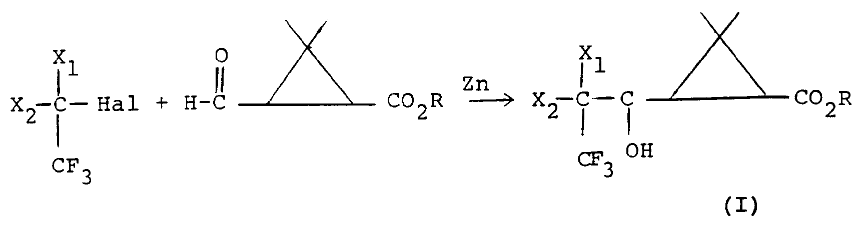

Voir à ce sujet Tetrahedron letters 1986, 27, p. 2139-2142, EP-A-187 642.

On vient de découvrir un nouveau procédé de préparation des composés de formule I.

L'invention a pour objet un procédé de préparation des composés de formule (I) : 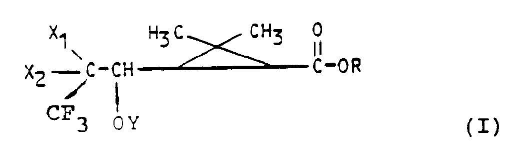
dans laquelle X₁ et X₂ identiques ou différents l'un de l'autre représentent un atome d'halogène, Y représente un radical choisi dans le groupe constitué par
- les radicaux SO₂alc₁, alc₁ représentant un radical alcoyle linéaire ou ramifié, saturé ou insaturé éventuellement substitué par un ou plusieurs groupements fonctionnels ;
- les radicaux SO₂Aᵣ, Aᵣ représentant un radical aryle renfermant jusqu'à 14 atomes de carbone, éventuellement substitué par un ou plusieurs groupements fonctionnels :
- les radicaux 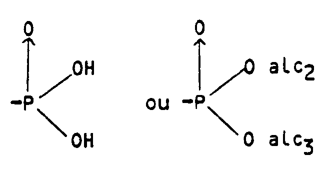 alc₂ et alc₃ identiques ou différents l'un de l'autre représentant un radical alcoyle linéaire ou ramifié saturé ou insaturé renfermant jusqu'à 8 atomes de carbone, éventuellement substitué par un ou plusieurs groupements fonctionnels ou alc₂ et alc₃
   pouvant former avec le radical 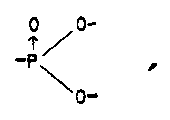 un cycle 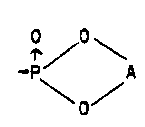 A représentant une chaîne carbonée linéaire ou ramifiée, saturée ou insaturée éventuellement substituée par un ou plusieurs groupements fonctionnels et renfermant jusqu'à 6 atomes de carbone.
- les radicaux 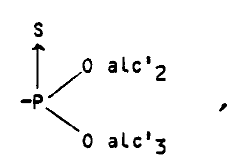 alc'₂ et alc'₃ représentant les mêmes valeurs que alc₂ et alc₃ ;
- le radical 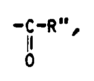 R'' représentant un radical alcoyle ou aryle éventuellement substitué par un ou plusieurs groupements fonctionnels et R représente un atome d'hydrogène, un radical alkyle linéaire ou ramifié, éventuellement substitué, renfermant jusqu'à 8 atomes de carbone, caractérisé en ce que l'on soumet un composé de formule II : 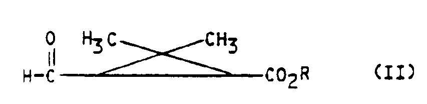 dans laquelle R conserve la même signification que précédemment à l'action d'un composé de formule V : 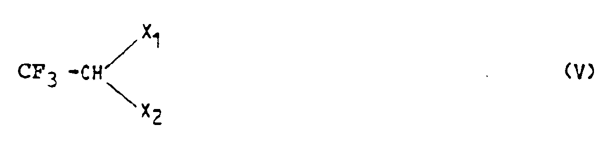 dans laquelle X₁ et X₂ conservent la même signification que précédemment en présence d'une base pour obtenir le composé de formule IV : 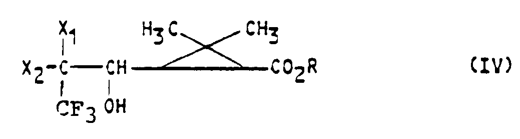 que l'on soumet à l'action d'un agent de sulfonylation ou de phosphonylation ou de thiophosphorylation ou d'acylation pour obtenir le composé de formule I correspondant : 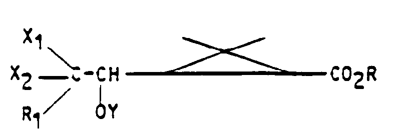 que l'on transforme éventuellement en acide correspondant que l'on soumet éventuellement à un agent d'estérification pour obtenir un nouveau composé de formule I.

Les composés de formule I présentent plusieurs centres d'asymétrie, les carbones en 1 et 3 du cyclopropane et les carbones en 1' et 2' de la chaîne latérale 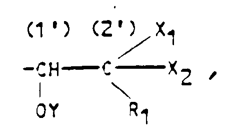
ils peuvent présenter également plusieurs centres d'asymétrie dans la partie R. L'invention a pour objet la préparation des différents stéréoisomères possibles ainsi que des mélanges de ces stéréoisomères.

Alc₁, alc₂, alc₃, alc'₂ et alc'₃ représentent de préférence un radical méthyle, éthyle, n-propyle, isopropyle, n-butyle, isobutyle, t-butyle.

Ar représente de préférence un radical phényle.

Lorsque alc₁, alc₂, alc₃, alc'₂, alc'₃ et Ar sont substitués, ils le sont de préférence par un ou plusieurs des substituants suivants : les atomes d'halogène, les radicaux CF₃, hydroxyle, carboxyle, amino, ammonium, les radicaux alkyle et alkoxy renfermant jusqu'à 4 atomes de carbone, par exemple les radicaux méthyle et méthoxy.

A représente de préférence une chaîne carbonée saturée renfermant de 1 à 4 atomes de carbone, éventuellement substituée par un ou plusieurs radicaux choisis dans le groupe suivant ; les radicaux alkyles renfermant de 1 à 5 atomes de carbone, les atomes d'halogène, les radicaux CF₃ ou les radicaux hydroxyles.

Lorsque R représente un radical alkyle, il s'agit de préférence du radical méthyle, éthyle, n-propyle, isopropyle, butyle, isobutyle, terbutyle.

L'invention a plus particulièrement pour objet un procédé de préparation des composés de formule I dans lesquels X₁ et X₂ représentent un atome de chlore, de brome ou d'iode, par exemple les composés dans lesquels X₁ et X₂ représentent un atome de brome.

Parmi les procédés de préparation préférés de l'invention, on peut citer le procédé de préparation des composés dans lesquels Y représente un radical SO₂alc₁, alc₁ représentant un radical alkyle ou alkényle renfermant jusqu'à 8 atomes de carbone par exemple un radical SO₂CH₃, ainsi que le procédé de préparation des composés de formule I dans lesquels Y représente un radical SO₂A_{r'}, Aᵣ conservant la même signification que précédemment.

Comme combinaisons préférées de X₁, X et CF₃, on peut citer : 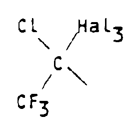
avec Hal₃ = Br, I

Dans un mode de réalisation préféré du procédé de l'invention :
- la base utilisée lors de la réaction du composé de formule II avec le composé de formule V est de préférence choisie dans le groupe constitué par les alcoolates alcalins, les hydrures alcalins et les hydroxydes alcalins. Comme bases tout particulièrement préférées, on peut citer la potasse, le méthylate ou le terbutylate de potassium ;
- L'agent de sulfonylation répond à la formule (A) 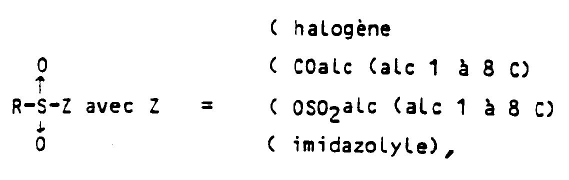
- L'agent de phosphonylation répond à la formule (B) 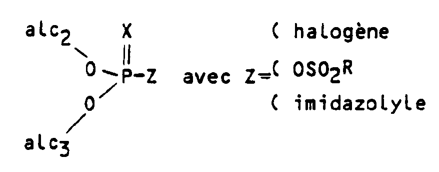 X représent eun atome d'oxygène ou de soufre et alc₂ et alc₃ conservent la même signification que dans la définition des produits de formule (I) ;
- l'agent d'acylation répond à la formule 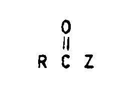 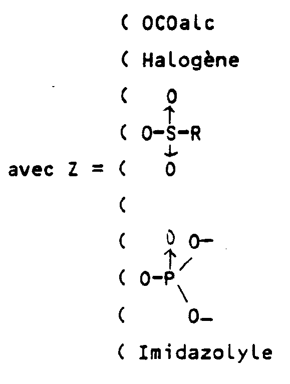
- l'agent de formule I peut être préparé en faisant réagir un ester de formule I avec un agent d'hydrolyse acide, par exemple l'acide paratoluènesulfonique, l'acide sulfurique, l'acide acétique ;
- L'agent d'estérification est un alcool, l'estérification étant réalisée selon les méthodes classiques.

Les exemples suivants illustrent l'invention sans toutefois la limiter :

### Exemple 1 : Acide [1R-[1-alpha, 3-alpha-(RS*,RS*)]] 2,2-diméthyl 3-[2-trifluorométhyl 2-bromo 2-chloro 1-méthyl sulfonyl éthyl] cyclopropane carboxylique.

### Stade A : [1R-[1-alpha, 3-alpha(RS*, RS*))] 2,2-diméthyl 3-[2-trifluorométhyl 2-bromo 2-chloro 1-méthyl hydroxyéthyl] cyclopropane carboxylique.

A une solution de 5 g de 1R,cis 2,2-diméthyl 3-formyl cyclopropane carboxylate de 1,1-diméthyléthyle dans 50 cm3 de THF et 3 cm3 d'halothéne (CF₃CHClBr) sont additionnés lentement à -70◊C, 3,1 g de terbutylate de potassium dans 20 cm3 de THF. Après un contact suffisant à cette température, le milieu est versé sur une solution aqueuse de PO₄H₂Na. On extrait au chlorure de méthylène, lave à l'eau, avec une solution saturée de chlorure de sodium. On sèche et amène à sec sous pression réduite. On obtient un produit que l'on chromatographie sur silice en éluant par le mélange hexane-éther isopropylique (8-2). On obtient ainsi 6,83 g de produit recherché.

### Stade B : [1R-[1-alpha, 3-alpha-(RS*RS*)]] 2,2-diméthyl 3-[2-trifluoro méthyl 2-bromo 2-chloro 1-méthyl sulfonyloxy éthyl] cyclopropane carboxylate de 1,1-diméthyléthyle.

On ajoute à -10◊C, 8 cm3 de triéthylamine dans un mélange de 6,74g de produit préparé au stade précédent, 40 cm3 de chlorure de méthylène et 4,3 cm3 de chlorure de mésyle. On agite pendant une heure au bain de glace, on décante, lave la phase organique à l'eau et à l'aide d'une solution saturée de chlorure de sodium, on sèche et amène à sec sous pression réduite. On obtient ainsi 9g d'un produit que l'on chromatographie sur silice en éluant par le mélange hexane-éther isopropylique (8-2). On obtient ainsi 5,67g de produit recherché fondant à 100◊C.

### Stade C : Acide [1R-[1-alpha, 3-alpha-(RS*,RS*)]] 2,2-diméthyl 3-[2-trifluorométhyl 2-bromo 2-chloro 1-méthyl sulfonyl éthyl] cyclopropane carboxylique.

On introduit à 40◊c 30mg d'acide paratoluène sulfonique dans une solution renfermant 1g du produit préparé au stage précédent et 10cm3 de chlorure de méthylène. On chauffe le mélange réactionnel pendant 5 heures à 40◊C. On verse sur la glace. On décante, lave la phase organique à l'eau, la sèche sur sulfate de sodium et amène à sec sous pression réduite. On obtient 860mg de produit recherché fondant à 138°C.

### Exemple 2: 1R (1-alpha, 3-alpha (RS*,RS*) 3-(2-bromo 2-chloro 1-[diéthoxy phosphoryloxy] 3,3,3-trifluoropropyl) 2,2-diméthyl cyclopropane carboxylate de 1,1-diméthyléthyle.

1,3 g de terbutylate de potassium dissous dans 10 cm3 de tétrahydrofuranne est introduit lentement à -60◊C dans une solution de 2 g de 1R (1-alpha, 3-alpha) 3-formyl 2,2-diméthyl cyclopropane carboxylate de 1,1-diméthyléthyle, 1,2 cm3 d'halothane (CF₃CHBrCl) dans 20 cm3 de tétrahydrofuranne. Après avoir agité 15 minutes à -60◊C une solution de 1,6 cm3 de chlorure de diéthyle phosphate dans 4 cm3 de tétrahydrofuranne, est ajoutée goutte à goutte. Après 30 minutes de contact à -60◊C, on verse le mélange sur une solution aqueuse de phosphate mono potassique. L'extraction au chlorure de méthylène permet d'isoler une huile qui est purifiée par chromatographie sur silice. Sont ainsi isolés 2,1 g de produit attendu.

## Revendications

1. Procédé de préparation des composés de formule (I) : 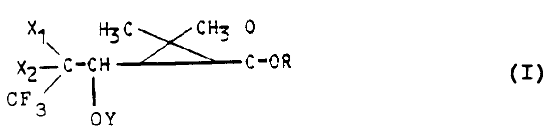 dans laquelle X₁ et X₂ identiques ou différents l'un de l'autre représentent un atome d'halogène, Y représente un radical choisi dans le groupe constitué par :
- les radicaux SO₂alc₁, alc₁ représentant un radical alcoyle linéaire ou ramifié, saturé ou insaturé éventuellement substitué par un ou plusieurs groupements fonctionnels ;
- les radicaux SO₂Aᵣ, Aᵣ représentant un radical aryle renfermant jusqu'à 14 atomes de carbone, éventuellement substitué par un ou plusieurs groupements fonctionnels ;
- les radicaux 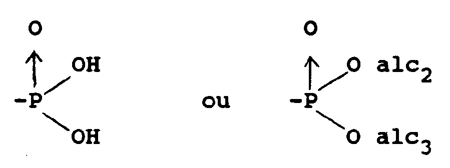 alc₂ et alc₃ identiques ou différents l'un de l'autre représentant un radical alcoyle linéaire ou ramifié saturé ou insaturé renfermant jusqu'à 8 atomes de carbone, éventuellement substitué par un ou plusieurs groupements fonctionnels ou alc₂ et alc₃
pouvant former avec le radical 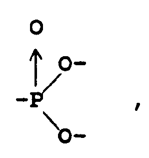 un cycle 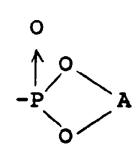 A représentant une chaîne carbonée linéaire ou ramifiée, saturée ou insaturée éventuellement substituée par un ou plusieurs groupements fonctionnels et renfermant jusqu'à 6 atomes de carbone ;
- les radicaux 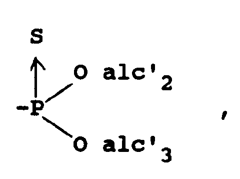 alc'₂ et alc'₃ représentant les mêmes valeurs que alc₂ et alc₃ ;
- le radical 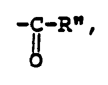 R'' représentant un radical aryle et R représente
- soit un atome d'hydrogène,
- soit un radical alkyle, linéaire ou ramifié, éventuellement substitué renfermant de 1 à 8 atomes de carbone, caractérisé en ce que l'on soumet un composé de formule II : 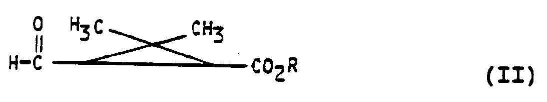
dans laquelle R conserve la même signification que précédemment à l'action d'un composé de formule V : 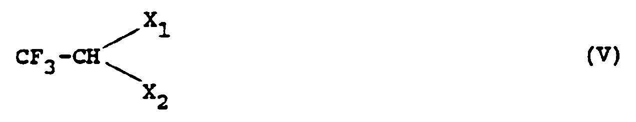 dans laquelle X₁ et X₂ conservent la même signification que précédemment en présence d'une base pour obtenir le composé de formule IV : 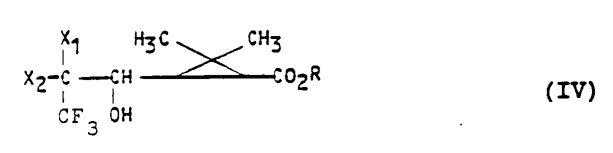 que l'on soumet à l'action d'un agent de sulfonylation ou de phosphonylation ou de thiophosphorylation ou d'acylation pour obtenir le composé de formule I correspondant : 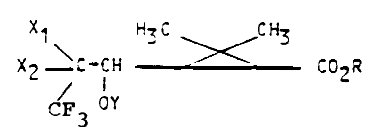 que l'on transforme éventuellement en acide correspondant que l'on soumet éventuellement à un agent d'estérification pour obtenir un nouveau composé de formule I.

2. Procédé de préparation des composés de formule I tels que définis à la revendication 1 dans lesquels X₁ et X₂ représentent un atome de chlore, de brome ou d'iode.

3. Procédé de préparation des composés de formule I tels que définis à la revendication 2 dans lesquels X₁ et X₂ représentent un atome de brome.

4. Procédé de préparation des composés de formule I tels que définis à l'une quelconque des revendications 1 à 3 dans lesquels Y représente un radical SO₂alc₁, alc₁ représentant un radical alkyle ou alkényle renfermant jusqu'à 8 atomes de carbone.

5. Procédé de préparation des composés de formule I tels que définis à la revendication 4 dans lesquels Y représente un radical SO₂CH₃.

6. Procédé de préparation des composés de formule I tels que définis à l'une quelconque des revendications 1 à 3 dans lesquels Y représente un radical SO₂Ar, Ar conservant la même signification que précédemment.

## Patentansprüche

1. Verfahren zur Herstellung von Verbindungen der Formel (I): 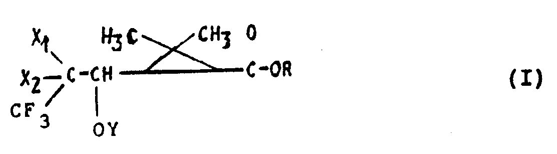 worin X₁ und X₂, die identisch oder voneinander verschieden sind, ein Halogenatom bedeuten, Y einen Rest bedeutet, ausgewählt aus der Gruppe bestehend aus:
- den Resten SO₂alc₁, wobei alc₁ einen geraden oder verzweigten, gesättigten oder ungesättigten Alkylrest darstellt, der gegebenenfalls durch eine oder mehrere funktionelle Gruppen substituiert ist;
- den Resten SO₂Aᵣ, wobei Aᵣ einen Arylrest darstellt, der bis zu 14 Kohlenstoffatome enthält, gegebenenfalls substituiert durch eine oder mehrere funktionelle Gruppen;
- den Resten 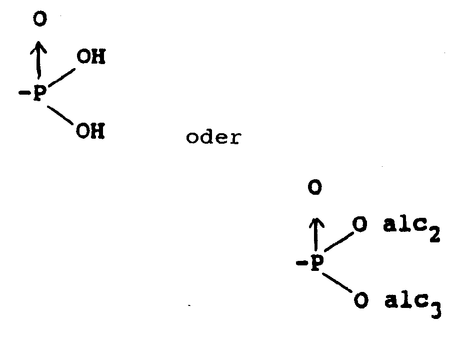 wobei alc₂ und alc₃, die identisch oder voneinander verschieden sind, einen geraden oder verzweigten, gesättigten oder ungesättigten Alkylrest mit bis zu 8 Kohlenstoffatomen bedeutet, der gegebenenfalls durch eine oder mehrere funktionelle Gruppen substituiert ist,
oder alc₂ und alc₃ können mit dem Rest 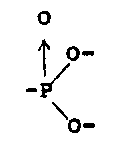 einen Zyklus 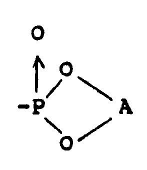 bilden, wobei A eine gerade oder verzweigte, gesättigte oder ungesättigte Kohlenstoffkette darstellt, die gegebenenfalls durch eine oder mehrere funktionelle Gruppen substituiert ist und bis zu 6 Kohlenstoffatome enthält;
- den Resten 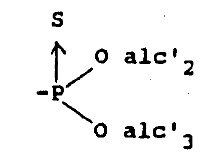 wobei alc'₂ und alc'₃ die gleichen Bedeutungen haben wie alc₂ und alc₃;
- dem Rest 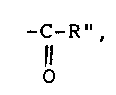 wobei R'' einen Arylrest bedeutet und R bedeutet
- entweder ein Wasserstoffatom
- oder einen geraden oder verzweigten, gegebenenfalls substituierten Alkylrest mit 1 bis 8 Kohlenstoffatomen, dadurch gekennzeichnet, daß man eine Verbindung der Formel II: 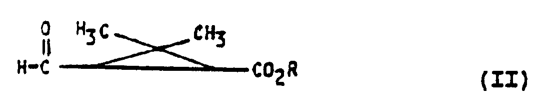
worin R die gleiche Bedeutung wie vorstehend hat, der Einwirkung einer Verbindung der Formel V: 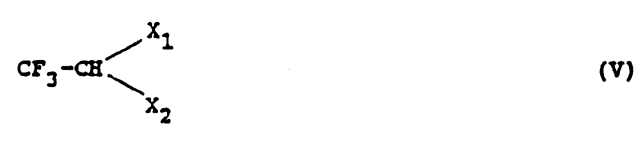 worin X₁ und X₂ die gleiche Bedeutung wie vorstehend haben, in Gegenwart einer Base unterwirft, um die Verbindung der Formel IV: 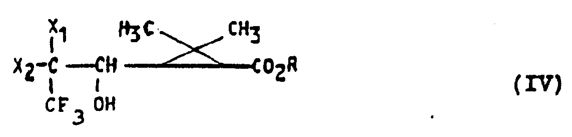 zu erhalten, die man der Einwirkung eines Sulfonylierungs- oder Phosphonylierungs- oder Thiophosphorylierungs- oder Acylierungsmittels unterwirft, um die entsprechende Verbindung der Formel I: 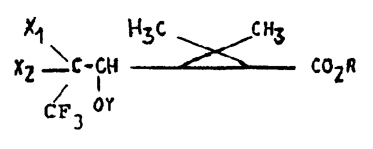 zu erhalten, die man gegebenenfalls in die entsprechende Säure überführt, die man gegebenenfalls einem Veresterungsmittel unterwirft, um eine neue Verbindung der Formel I zu erhalten.

2. Verfahren zur Herstellung der Verbindungen der Formel I, wie sie in Anspruch 1 definiert sind, worin X₁ und X₂ ein Chlor-, Brom- oder Jodatom bedeuten.

3. Verfahren zur Herstellung der Verbindungen der Formel I, wie sie in Anspruch 2 definiert sind, worin X₁ und X₂ ein Bromatom bedeuten.

4. Verfahren zur Herstellung der Verbindungen der Formel I, wie sie in einem der Ansprüche 1 bis 3 definiert sind, worin Y einen Rest SO₂alc₁ bedeutet, wobei alc₁ einen Alkyl- oder Alkenylrest mit bis zu 8 Kohlenstoffatomen bedeutet.

5. Verfahren zur Herstellung.der Verbindungen der Formel I, wie sie in Anspruch 4 definiert sind, worin Y einen Rest SO₂CH₃ darstellt.

6. Verfahren zur Herstellung der Verbindungen der Formel I, wie sie in einem der Ansprüche 1 bis 3 definiert sind, worin Y einen Rest SO₂Ar bedeutet, wobei Ar die gleiche Bedeutung wie vorstehend hat.

## Claims

1. Preparation process for compounds of formula (I): 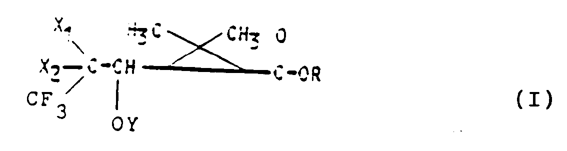 in which X₁and X₂, identical to or different from each other, represent a halogen atom, Y represents a radical chosen from the group constituted by:
- SO₂alk₁ radicals, alk₁ representing a saturated or unsaturated, linear or branched alkyl radical optionally substituted by one or more functional groups;
- SO₂Aᵣ radicals, Aᵣ representing an aryl radical containing up to 14 carbon atoms, optionally substituted by one or more functional groups;
- the radicals 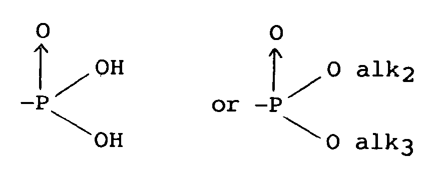 alk₂ and alk₃, identical to or different from each other, representing a saturated or unsaturated, linear or branched alkyl radical containing up to 8 carbon atoms, optionally substituted by one or more functional groups, or alk₂ and alk₃
being able to form with the radical 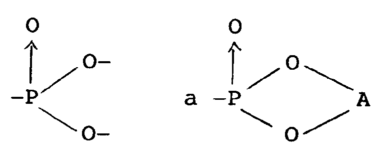 ring, A representing a saturated or unsaturated, linear or branched carbon-containing chain optionally substituted by one or more functional groups and containing up to 6 carbon atoms;
- the radicals 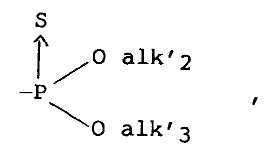 alk'₂ and alk'₃ representing the same values as alk₂ and alk₃;
- the 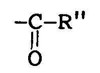 radical, R'' representing an aryl radical and R represents
- either a hydrogen atom,
- or an optionally substituted linear or branched alkyl radical containing 1 to 8 carbon atoms, characterized in that a compound of formula II: 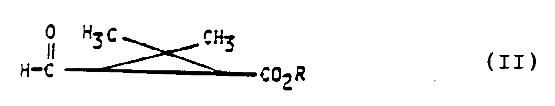
in which R keeps the same meaning as previously, is subjected to the action of a compound of formula V: 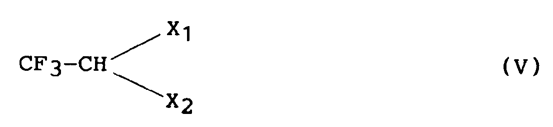 in which X₁ and X₂ keep the same meaning as previously, in the presence of a base, in order to obtain the compound of formula IV: 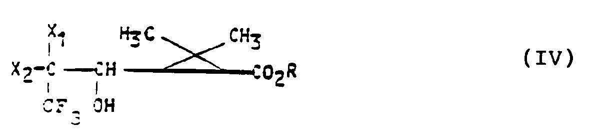 which is subjected to the action of a sulphonylation or phosphonylation or thiophosphorylation or acylation agent in order to obtain the corresponding compound of formula I: 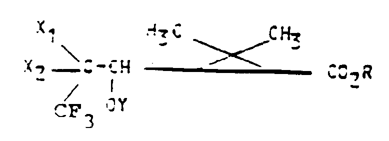 which is optionally converted into the corresponding acid which is optionally subjected to an esterification agent in order to obtain a new compound of formula I.

2. Preparation process for compounds of formula I as defined in claim 1 in which X₁ and X₂ represent a chlorine, bromine or iodine atom.

3. Preparation process for compounds of formula I as defined in claim 2 in which X₁ and X₂ represent a bromine atom.

4. Preparation process for compounds of formula I as defined in any one of claims 1 to 3 in which Y represents an SO₂alk₁ radical, alk₁ representing an alkyl or alkenyl radical containing up to 8 carbon atoms.

5. Preparation process for compounds of formula I as defined in claim 4 in which Y represents an SO₂CH₃ radical.

6. Preparation process for compounds of formula I as defined in any one of claims 1 to 3 in which Y represents an SO₂Ar radical, Ar keeping the same meaning as previously.
